# EUROPEAN PATENT APPLICATION

(11) **EP 1 938 804 A1**
(43) Date of publication of application: **02.07.2008**
(21) Application number: 06126964.3
(22) Date of filing: 22.12.2006
(51) Int. Cl.: A61K 9/16, A61K 9/14, A61K 31/55, A61K 31/4468

(54) **Pharmaceutical formulation comprising neurokinin antagonist**

(71) Applicant: Novartis AG, 4056 Basel (CH)
(72) Inventor: The designation of the inventor has not yet been filed
(74) Representative: McLean, Craig Sutherland

(57) **Abstract**

Pharmaceutical compositions in the form of a solid dispersion comprising (4R)-4-[N'-methyl-N'-(3,5-bistrifluoromethyl-benzoyl)-amino]-4-(3,4-dichlorobenzyl)-but-2-enoic acid N-[(R)-cpsilon-caprolactam-3-yl]-amidc or a pharmaceutically acceptable salt or solvate thereof and a carrier. Such compositions are useful to treat patients who have functional motility disorders of the viscera, especially irritable bowel syndrome or functional dyspepsia, or urinary bladder disorders, especially urinary incontinence.

## Description

The present invention relates to novel pharmaceutical compositions comprising a neurokinin antagonist, in particular an acylaminoalkenylene amide.

More specifically, the present invention relates to a solid dispersion comprising (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a salt or solvate thereof, which is also known as N-[(*R*,*R*)-(*E*)-1-(3,4-dichlorobenzyl)-3-(2-oxoazepan-3-yl) -carbamoyl]-allyl-N-methyl-3,5-bis (trifluoromethyl)-benzamide and has the chemical structure of formula I

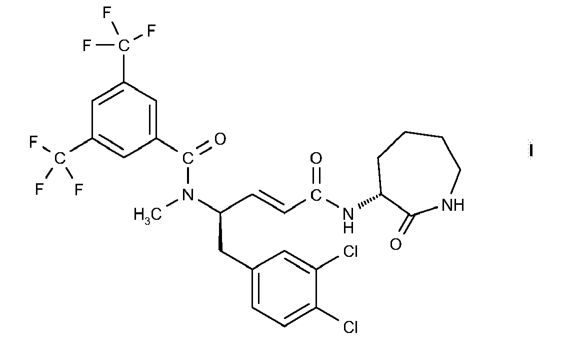

The compound of formula I, especially the semihydrate thereof, is useful in the treatment of a number of conditions associated with neurokinins (substance P, neurokinin A, neurokinin B), for example (i) functional motility disorders of the viscera including those associated with visceral hypersensitivity and/or altered motor responses (including electrolyte/water secretion), for example functional bowel disorders and functional gastrointestinal disorders, such as irritable bowel syndrome (IBS), constipation, diarrhoea, functional dyspepsia, gastro-oesophageal reflux disease, functional abdominal bloating, and functional abdominal pain, other conditions associated with visceral hypersensitivity such as post-operative visceral pain, visceral smooth muscle spasms, and irritable bladder and other functional bowel disorders (not necessarily associated with visceral hypersensitivity or abnormal motor responses) as described in international patent application WO 03/66062, or (ii) urinary bladder disorders such as urinary incontinence, which may be for example, urge incontinence, stress incontinence, mixed urge/stress incontinence, or neurogenic incontinence (unstable detrusor and detrusor hyperreflexia, decreased bladder compliance, sensory urgency, bladder-related visceral pain) as described in international patent application WO 05/39563. The invention is of particular importance for the treatment of irritable bowel syndrome (IBS), especially diarrhoea- predominant IBS, and functional dyspepsia (FD). A method for the preparation of the compound of formula I is described in international patent application WO 98/07694, the contents of which are incorporated herein by reference.

This compound presents highly specific difficulties in relation to administration generally and the preparation of galenic compositions in particular, including in particular problems of drug bioavailability and variability in inter- and intra-patient dose response, necessitating development of a non-conventional dosage form.

It has now been found that (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide can be formulated as a solid dispersion, giving an advantageous stability and bioavailability profile.

In a first aspect the present invention provides a pharmaceutical composition in the form of a solid dispersion comprising (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a pharmaceutically acceptable salt or solvate thereof and a carrier.

The term "solid dispersion" as used herein is understood to mean a system in a solid state that comprises at least two components, wherein one component is dispersed substantially evenly throughout the other component or components. This includes solid or glassy solutions i.e. the dispersion of the components is such that the system is chemically and physically uniform or homogenous throughout or consists of one phase (as defined in thermodynamics). A "solid dispersion" may comprise a co-precipitate or co-melt of an active ingredient and a carrier medium, wherein the active ingredient is dispersed substantially evenly throughout the carrier system. The active ingredient may be present in a glassy amorphous state or in fine crystalline dispersed form. It may be present as a mixture of amorphous and crystalline forms. The term solid dispersion may also encompass systems which consist of more than one phase. That means the active ingredient may exist in multiple phases such as a pure drug phase and/or in drug rich phases and/or drug poor phase. In such multi-phase systems there also may be phases without drug existing. The term "solid dispersion" as used herein also encompasses eutectic mixtures of the active ingredient.

Throughout this specification and in the claims that follow, unless the context requires otherwise, the word "comprise", or variations such as "comprises" or "comprising", should be understood to imply the inclusion of a stated integer or step or group of integers or steps but not the exclusion of any other integer or step or group of integers or steps.

The compound of formula I includes all pharmaceutically acceptable isotopically-labelled forms thereof wherein one or more atoms are replaced by atoms having the same atomic number, but an atomic mass or mass number different from the atomic mass or mass number usually found in nature. Examples of isotopes suitable for inclusion in the compounds of the invention include isotopes of hydrogen e.g. ²H and ³H, carbon e.g. ¹¹C, ¹³C and ¹⁴C, chlorine e.g. ³⁶Cl, fluorine e.g. ¹⁸F, iodine e.g. ¹²³I and ¹²⁵I, nitrogen e.g. ¹³N and ¹⁵N, oxygen e.g. ¹⁵O, ¹⁷O and ¹⁸O, and sulfur e.g. ³⁵S.

Certain isotopically-labelled forms of the compound of formula I, for example those incorporating a radioactive isotope, are useful in drug and/or substrate tissue distribution studies. The radioactive isotopes tritium (³H) and carbon-14 (¹⁴C) are particularly useful for this purpose in view of their ease of incorporation and ready means of detection. Substitution with heavier isotopes such as deuterium (²H) may afford certain therapeutic advantages that result from greater metabolic stability, for example increased *in vivo* half-life or reduced dosage requirements, and hence may be preferred in some circumstances. Substitution with positron emitting isotopes, such as ¹¹C, ¹⁸F, ¹⁵O, and ¹³N can be useful in Positron Emission Topography (PET) studies for examining substrate receptor occupancy.

Isotopically-labelled forms of the compound of formula I can generally be prepared by conventional techniques known to those skilled in the art or by processes analogous to those described in the accompanying examples using an appropriate isotopically-labelled reagent in place of the non-labelled reagent previously used.

In a second aspect the present invention provides a method of treating a subject suffering from a disorder treatable with a neurokinin antagonist comprising administering a therapeutically effective amount of a pharmaceutical composition in the form of a solid dispersion comprising (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a pharmaceutically acceptable salt or solvate thereof and a carrier to a subject in need of such treatment.

In a third aspect the present invention provides a method of treatment of a subject suffering from (i) gastrointestinal disorders, especially diarrhoea-predominant IBS and functional dyspepsia, (ii) urinary bladder disorders, especially urge incontinence, or (iii) respiratory diseases, especially asthma. comprising administering a therapeutically effective amount of a pharmaceutical composition in the form of a solid dispersion comprising (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a pharmaceutically acceptable salt or solvate thereof and a carrier to a subject in need of such treatment.

The pharmaceutical compositions of the present invention may be prepared by a number of methods.

In a first method, the drug substance or active ingredient is dissolved in a solvent or a solvent mixture with one or more excipients, including a carrier substance. Some or all excipients may also be present in the solvent or solvent mixture dissolved or in a suspended or swollen state. The resulting feed solution or suspension may be dried by spray drying to form a solid dispersion. The term spray drying refers to processes which involve the atomization of the feed suspension or solution into small droplets and rapidly removing solvent from the mixture in a processor-chamber where there is a strong driving force for the evaporation of the solvents (i.e. hot dry gas or partial vacuum or combinations thereof).

In a second method, the feed solution or suspension is atomized and dried in the processor chamber of a spray dryer or a fluidized spray drier to form primary particles which are subsequently agglomerated in a fluid bed.

In a third method, the feed solution or suspension is dried by being atomized into a processor chamber of a fluid bed-type processor or a pan-coater which is charged with inert filler material. During drying the filler becomes agglomerated and/or coated and/or layered by the solid dispersion.

In a fourth method the solid dispersion is prepared by melting the drug substance and/or the carrier. Atomization and resodification is done in a fluid bed processor.

In a fifth method, the feed solution or suspension is dried and/or dried and chopped down at elevated temperature and/or partial vacuum for example in a rotavapor-like processor or paddle dryer-type processors.

In a sixth method, solid dispersions are prepared by melting the drug substance and/or the carrier using a melt extruder.

In a seventh method solid dispersions are prepared by melting drug substance and/or the carrier in presence of a filler material wherein the particles are agglomerated and/or coated using a melt extruder.

In an eighth method solid dispersions are prepared by dissolving the drug substance using a solvent or a low melting material (for example: a polymer, plasticizer, wax, surfactant) and then mixing it into a carrier using a melt extruder.

In a ninth method solid dispersions are prepared by dissolving the drug substance using a solvent and drying it under reduced pressure, for example by using sealing elements before and after a vacuum port in the melt extruder.

In a tenth method solid dispersions are prepared by precipitation e.g. by rapid mixing of the feed solution or suspension with CO₂, or an other non-solvent.

Suitable solvents for the solvent evaporation methods are alcohols such as ethanol, methanol, n-propanol, iso-proponal and butanol. Ketones such as acetone and methyl ethylketone and various other solvents like methylene chloride. Mixtures of these solvents may also be used. The solvent or solvent mixture may also contain up to 40% water in order to increase the swelling of selected polymeric carrier in the feed and thus to finally optimize the dispersion grade of the product (= solid dispersion).

The solid dispersions may be further processed into tablet, or capsule form or may be processed into multiparticular systems e.g. minitablets or pour-into mouse granules or oral powders for constitution.
In one embodiment of the present invention there is provided a solid dispersion comprising (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a pharmaceutically acceptable salt or solvate thereof, and a polymeric carrier.

Suitable polymeric carriers include water-soluble polymers, preferably a cellulose derivative such as hydroxypropylmethylcellulose (HPMC), hydroxypropylcellulose (HPC) or polyvinylpyrrolidone (PVP). Good results may be obtained using HPMC with a low apparent dynamic viscosity, e.g. from about 0.01 cps to about 100 cps as measured at 20°C for a 2% by weight aqueous solution, e.g. from about 0.01 cps to about 50 cps, preferably from about 0.01 cps to about 20 cps, for example HPMC 3 cps. HPMC is well-known and described, for example, in the Handbook of Pharmaceutical Excipients, Second Edition, Pharmaceutical Society of Great Britain and American Pharmaceutical Association, 1994, pages 229 to 232, the contents of which are incorporated herein by reference. HPMC, including HPMC 3 cps, is available commercially under the trade mark Pharmacoat® 603 from the Shinetsu company. PVP is available, for example, under the trade mark Povidone® (Handbook of Pharmaceutical Excipients, pages 392-399), and a PVP having an average molecular weight between about 8,000 and about 50,000 Daltons is preferred, e.g. PVP K30.

Suitable polymer carriers also include polymers which show a pH-dependant swelling and thus may slow down or avoid drug release from the solid dispersion in gastric environment but swell or dissolve in intestinal juice. Examples of these polymers that are applied for enteric coating of tablets include the cellulose derivatives hydroxyl-propyl methylcellulose acetate succinate (HPMCAS), e.g. Aqoat MF or HE and hydroxypropylmethylcellulose phthalate (e.gHPMCP-.HP50 or HPMC-HP55). Suitable polymer carriers also include a copolymer formed from monomers selected from the group consisting of methacrylic acid, methacrylic acid esters, acrylic acid and acrylic acid esters, e.g. as those known and commercially available under the trade mark Eudragit® from R 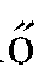 hm Pharma GmbH. An especially preferred polymer is the 1:1 or 1:2 copolymer formed from monomers selected from the group consisting of methacrylic acid and methacrylic acid lower alkyl esters, such as the 1:1 or 1:2 copolymer formed from methacrylic acid and methyl methacrylate. The 1:1 copolymers are available under the trade mark Eudragit® L, the 1:2 copolymers are available under the trade mark Eudragit® S. A particularly preferred polymer is the 1:1 copolymer of methacrylic acid and the acrylic acid ethyl ester as known and commercially available under the trade mark Eudragit®L 100-55. The enteric polymers may also be used in combination with polymers that are non-resistant against gastric juice (e.g. HPMC or PVP) in order to allow to optimize locally available drug concentrations in the GIT and thus optimize the bioavailability.

In another embodiment the polymeric carrier comprises:
(i) hydroxypropylcellulose (HPC) or a derivative thereof. Examples of HPC derivatives include those having low dynamic viscosity in aqueous media, e.g. water, e.g. from about 0.01 cps to abut 400 cps, e.g. from about 0.01 cps to about 150 cps as measured in a 2% aqueous solution at 25°C. Preferred HPC derivatives have a low degree of substitution, and an average molecular weight e.g. between about 5000 and about 200,000 Daltons, e.g. between about 50,000 and about 150,000 Daltons. Examples of HPC available commercially include Klucel® LF, Klucel® EF and Klucel® JF from the Aqualon company; and Nisso® HPC-L available from Nippon Soda Ltd;
(ii) a cyclodextrin, for example, a beta-cyclodextrin or an alpha-cyclodextrin. Examples of suitable beta-cyclodextrins include methyl-beta-cyclodextrin; dimethyl-beta-cyclodextrin; hyrdroxypropyl-beta-cyclodextrin; glycosyl-beta-cyclodexterin; maltosyl-beta-cyclo-dextrin; sulfo-beta-cyclodextrin; sulfo-alkylethers of beta-cyclodextrin, e.g. sulfo-C[1-4]-alkyl ethers. Examples of alpha-cyclodextrins include glucosyl-alpha-cyclodextrin and maltosyl-alpha-cyclodextrin;
(iii) a polyethylene glycol (PEG). Examples include PEGs having an average molecular weight between 1000 and 9000 Daltons, e.g. between about 1800 and 7000, for example PEG 2000, PEG 4000 or PEG 6000 (Handbook of Pharmaceutical Excipients, pages 355-361);
(iv) a polymethycrylate (not just gastric-resistant); and
(v) a polyvinyl alcohol polymer and co-polymers thereof with PVP or other polymers.

In another embodiment the non polymeric carrier comprises low molecular weight substances which from an amorphous glass such as saccharoses e.g. mannitol, sorbitol or substances like urea.

The carrier may further comprise one or more surfactants or wetting agents, for example a non-ionic, ionic, anionic or amphoteric surfactant. Examples of suitable surfactants/wetting agents include polyoxyethylene-polyoxypropylene co-polymers and block co-polymers known, for example, under the trade marks Pluronic® or Poloxamer®, polyoxyethylene-sorbitan-fatty acid esters including mono- and tri-lauryl, palmityl, stearyl and oleyl esters of the type known under the trade name Tween®, polyoxyethylene fatty acid esters including polyoxyethylene stearic acid esters of the type known under the trade name Myrj®, polyoxyethylene alkyl ethers known under the trade mark Brij®, sodium alkyl sulfates and sulfonates, and sodium alkyl aryl sulfonates, water soluble tocopheryl polyethylene glycol succinic acid esters (TPGS), polyglycerol fatty acid esters, alkylene polyol ethers or esters, polyethylene glycol glyceryl fatty acid esters, sterols and derivatives thereof, transesterified, polyoxyethylated caprylic-capric acid glycerides, sugar fatty acid esters, PEG sterol ethers, phospholipids, salts of fatty acids, fatty acid sulfates and sulfonates, salts of fatty acids, fatty acid sulfates and sulfonates, medium or long-chain alkyl, e.g. C₆-C₁₈, ammonium salts, bile acid or salt thereof; for example cholic acid, glycolic acid or a salt, e.g. sodium cholate and polyoxyethylene mono esters of a saturated C₁₀ to C₂₂ fatty acid.

In a further embodiment, the present invention provides a pharmaceutical composition in the form of a solid dispersion comprising(4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a pharmaceutically salt thereof, a polymeric carrier and a pH modifiers such as acids, bases or buffers which may retard or enhance the swelling of the polymeric carrier in the GIT and thus may allow to target the drug release into the desired location. In addition conventional additives such as fillers, disintegrants, anti-oxidants, binders or anti-sticking agents may be part of the solid dispersion itself. When these additives are included as part of the dispersion they may be dissolved or suspended or mixed into the feed from which the solid dispersion is formed or alternatively be used as starter material (filler) in e.g. pan-coaters or fluid bed processors.

Suitable pH modifiers include but are not limited to citric acid., lactic acid succinic acids and bases like sodium acetate, calcium oxide, sodium hydroxide and buffer systems.
Suitable filler (or diluent) materials include but are not limited to, water-soluble or water-insoluble compounds such as lactose, sucrose, amylose, dextrose, mannitol and inositol, xylitol, microcrystalline cellulose, but preferably lactose, mannitol or microcrystalline cellulose. Microcrystalline cellulose is available commercially under the trade mark Avicel®, Pharmacel®, Emcocell®, Vivapur®, preferably Avicel®, available e.g. from FMC Corporate (Handbook of Pharmaceutical Excipients, pages 84-87). The fillers may also be used in form of rough more or less spheric particles like Pellets ; Cellets, , Celsphere® which are preferred starting materials for coating and layering technology. Suitable filler and anti-sticking agents also include colloidal Slicon Dioxide like Aerosil®200 or Talc.

Examples of pharmaceutically acceptable disintegrants include, but are not limited to, starches; clays; celluloses; alginates; gums; cross-linked polymers, e.g. cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum.

The pharmaceutical compositions of the present invention may include additional excipients that are commonly employed in the preparation of dosage forms, such as disintegrants, lubricants, glidants, binders and fillers.

Examples of pharmaceutically acceptable lubricants and pharmaceutically acceptable glidants include, but are not limited to, colloidal silica, magnesium trisilicate, starches, talc, tribasic calcium phosphate, magnesium stearate, aluminium stearate, calcium stearate, magnesium carbonate, magnesium oxide, polyethylene glycol, powdered cellulose, glyceryl behenate, stearic acid, hydrogenated castor oil, glyceryl monostearate, and sodium stearyl fumarate.

Examples of pharmaceutically acceptable binders include, but are not limited to, starches; celluloses and derivatives thereof, e.g., microcrystalline cellulose, e.g., AVICEL PH from FMC (Philadelphia, PA), hydroxypropyl cellulose hydroxylethyl cellulose and hydroxyl-propylmethyl cellulose METHOCEL from Dow Chemical Corp. (Midland, MI); sucrose; dextrose; corn syrup; polysaccharides; and gelatin.

Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc.

The pharmaceutical compositions of the present invention may further include additives or ingredients, such as antioxidants (e.g. ascorbyl palmitate, butyl hydroxy anosole (BHA), butyl hydroxy toluene (BHT), tocopherols, propyl gallate and fumaric acid), antimicrobial agents, enzyme inhibitors, stabilizers (e.g. malonic acid), and/or preserving agents.

The active ingredient may be present in an amount by weight of the composition of about 0.01 % to about 80%; for example, in an amount by weight of about 0.01% to about 80%, 0.1% to about 70%, such as 1%% to 60%, for example 2%, 5%, 10%, 20%, 30%, 40%, 50%, or 60%.

The polymeric carrier may be present in an amount from about 0.1% to 99.99% by weight of the composition.

When a plasticizer or surfactant is present, it may generally be present in an amount of from about 0.01% to about 50%, for example from about 1% to about 30% by weight, e.g. 5% to 20% by weight of the composition.

When a disintegrant is present in the pharmaceutical composition, it may be generally be present in an amount from about 1% to about 30% by weight of the composition, from about 5 % to about 10% to about 20% by weight of the composition.

When a filler is present, it may generally be present in an amount of from about 0.01 to about 99% by weight of the composition, e.g. from about 0.5 to about 70% by weight, such as about 30%, 40% or 50% to about 60% by weight of the composition.

When a lubricant is present, it may generally be present in amounts from about 0.1% to about 5% by weight of the composition; whereas, the glidant, e.g., may be present in an amount from about 0.1% to about 10% by weight of the composition.

When additives, for example antioxidants, are present they may generally comprise about 0.05-5%, preferably 0.05-1% by weight of the composition.

When pH modifying agents is present they may generally comprise about 0.05-20% by weight of the composition.

When required, the solid dispersions of the invention are preferably compounded in unit dosage form, e.g. as a tablet, a capsule or a multi-particulate system such as granules or a powder, for administration. Where the composition is in unit dosage form, each unit dosage will suitably contain from 0.1 and 150 mg active agent, for example 0.1 mg, 1 mg, 5 mg, 10 mg, 15 mg, 25 mg, 50 mg, or 100 mg, e.g. between 5 and 100 mg of the active agent. Such unit dosage forms are suitable for administration 1 to 5 times daily depending upon the particular purpose of therapy, the phase of therapy and the like.

The dosage form used, e.g. a tablet, granules or powder may be coated, for example using an enteric coating. Suitable coatings may comprise but are not limited to cellulose acetate phthalate; hydroxypropylmethylcellulose phthalate; a polymethyacrylic acid copolymer, e.g. Eudragit L or Eudragit S; or hydroxyl-propyl methylcellulose acetate succinate, e.g. Aqoat MF or HF.

Unit doses of the pharmaceutical composition of the invention are preferably compartmentalised in a blister pack to avoid moisture uptake inducing crystal formation and negatively affecting the stability of the composition generally. This is particularly important when the composition contains hydroscopic substances such as certain disintegrants.

The pharmaceutical compositions of the invention exhibit especially advantageous properties when administered orally; for example in terms of consistency and high level of bioavailability obtained in standard bioavailability trials. Such trials are performed in animals, e.g. rats or dogs or healthy volunteers using chromatographic methods, e.g. HPLC.

The invention is illustrated in the following non-limiting examples.

### EXAMPLES

In these examples "Compound A" is micronised (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide semihydrate.

A control formulation comprising the components listed in Table 1 below is prepared as a powder blend in a hard gelatine capsule.

**TABLE 1**

| **Component** | **Mass (mg/HK)** |
|---|---|
| Compound A (active ingredient) | 20.0 |
| Lactose (filler) | 49.0 |
| Crospovidone (disintegrant) | 10.0 |
| Sodium lauryl sulfate (surfactant) | 20.0 |
| Aerosil 200 (glidant) | 0.5 |
| Magnesium stearate (lubricant) | 0.5 |

### Example 1

### Spray-dried solid dispersions

Solid dispersions 1A and 1C to 1K are manufactured in a Buechi-Minispary apparatus.

1A, 1D, 1E, 1F and solid dispersion 1G-1K are prepared by dissolving Compound A in a 1:1 mixture of acetone and ethanol (abs). For solid dispersion C a 1:1 mixture of acetone and ethanol 94% den. (= ethanol quality with water and isopropanol) is used.

When a clear solution has formed, the other components listed in Tables 2 and 3 are added into this mixture to give a feed-suspension or -solution. Typical solid to liquid ratios in these feeds are 1:5 to 1:7. The feed is atomized into a hot nitrogen gas atmosphere of a spray dryer to remove the solvents at a product-temperature around 90°C. The solid dispersion is collected as powder from the processor.

Solid dispersion 1B is prepared on the Pilot spray dryer Niro-Mobile Minor. A 1:1 mixture of acetone and ethanol 94% den. (= ethanol quality with water and isopropanol) is used.
When a clear solution has formed, HPMC is added into this mixture to give a feed-suspension. After adding of the polymer the feed is vigorously stirred for al least 1 hour. Subsequently it is passed through an IKA-mill (Pilot SN1102) for wet-milling. The resulting feed is atomized into the hot nitrogen of the spray drier to remove the solvents at a product-temperature of approx. 90-100°C. The dry solid dispersion is collected as powder on the cyclone discharge valve.

**TABLE 2**

| **Component** | **1A** | **1B** | **1C** | **1D** | **1E** | **1F** |
|---|---|---|---|---|---|---|
| Compound A (active ingredient) | 20% | 40% | 60% | 80% | 40% | 20% |
| HPMC-3cps (polymeric carrier) | 80% | 60% | 40% | 20% | 55% | 70% |
| Myrj 59 (su rfactant) | | | | | 5% | |
| Poloxamer 188 (surfactant) | | | | | | 10% |

**TABLE 3**

| **Component** | **1G** | **1H** | **1J** | **1K** |
|---|---|---|---|---|
| Compound A (active ingredient) | 80% | 40% | 60% | 20% |
| PVP K30 (polymeric carrier) | 20% | 60% | 40% | 80% |

Some of those solid dispersions are blended with the excipients listed in Table 4 or 5 and then the blended mixture is compressed into tablets (T) or filled into hard gelatin capsules (H).

**TABLE 4**

| **Component** | **1L** | **1M** | **1N** | **1P** |
|---|---|---|---|---|
| | Tablet | tablet | 2x tablets | tablet |
| Solid dispersion | 100 mg 1A (20%) | 50 mg 1B (40%) | 62.5 mg 1B (40%) | 166.65 mg 1C (60%) |
| Lactose (filler) | 210.8 mg | 175.5 mg | 156.25 mg | 274.35mg |
| Sodium carboxymethyl starch (disintegrant) | 80 mg | 18.75 mg | 25.0 mg | 50.0 mg |
| Colloidal silica (lubricant) | 6.0 mg | 3.75 mg | 3.75 mg | 4.0 mg |
| Magnesium stearate (lubricant) | 3.2 mg | 2.0 mg | 2.5 mg | 5.0 mg |

The mass of Compound A contained in tablets/capsules 1L, 1M, 1N and 1P is 20 mg, 20 mg, 25 mg and 100 mg respectively.

**TABLE 5**

| **Component** | **1Q** | **1R** | **1S** |
|---|---|---|---|
| | Tablet | Hard gel caps | tablet |
| Solid dispersion | 50mg 1E (40%) | 125 mg 1F (20%) | 166.7 mg 1J (60%) |
| Lactose (filler) | 175.5 mg | 125 mg | 275.3 mg |
| Sodium carboxymethyl starch (disintegrant) | 18.75 mg | - | 150.0 mg |
| Colloidal silica (lubricant) | 3.75 mg | - | 4.0 mg |
| Magnesium stearate (lubricant) | 2.0 mg | - | 4.0 mg |

The mass of Compound A contained in tablets/capsules 1Q, 1R and 1S is 20 mg, 25 mg and 100 mg respectively.

The dissolution rate of some of the tablets/capsules is determined giving the results shown in TABLE 6 below. In each case the tablet/capsule is added to a solution containing 1000 mL of 0.1N HCl and 0.3% Tween® 20, 50 rpm paddle, 37°C.

**TABLE 6**

| **Time** | **Control (%)** | **1L (%)** | **1N (%)** | **1Q (%)** | **1R (%)** |
|---|---|---|---|---|---|
| 20 min | 63.5 ± 10.8 | 80.2 ± 5.5 | n.d. | - | 91.2 ± 4.4 |
| 30 min | 69.4 ± 11.9 | 90 ± 3.7 | 98.6 ± 1.5 | 83 ± 1 | 100.9 ± 1.1 |
| 60 min | 74.4 ± 13.6 | 94 ± 0.3 | 101.2 ± 0.5 | 87 ± 0.5 | 101.2 ± 0.5 |

| | | | | | |
|---|---|---|---|---|---|
| Where n.d. = not determined | | | | | |

Table 6 shows that a significant increase in the dissolution rate of the solid dispersions is observed in comparison to the control.

Solid dispersions and tablets are stored at 25°C and 60% relative humidity in closed and open containers and at 40°C and 75% relative humidity in closed and open containers. However in each case no change in crystal structure is observed over 3 months as measured by differential scanning calorimetry (DSC) and X-ray powder diffraction (XRPD). This indicates that the solid dispersions remain amorphous even under accelerated conditions (i.e. 40°C/ 75% r.h. open).

### Example 2

### Solid dispersions manufactured in fluid bed processors

Solid dispersions 2A to 2D are prepared using the components listed in Table 7 below.

Compound A is dissolved in a 1:1 mixture of acetone and ethanol mixture to form a clear solution. Subsequently, the polymeric carrier is suspended into that mixture. Typical liquid to solid ratios are 1:5 to 1:7. An inert filler material (e.g. an Lactose-Aerosil blend) is fluidised in a warm process air stream in the lab scale fluidized bed processor (Aeromatic™) and the feed is atomized via a top spray two-fluid nozzle into the processor causing the filler to get agglomerated and coated by the solid dispersion while evaporating the solvents. Typical product temperatures are 20-50°C. The solid dispersion is collected from the product container of the processor as free-flowing granules.

**TABLE 7**

| **Component** | **2A** | **2B** | **2C** | **2D** | **2E** |
|---|---|---|---|---|---|
| Compound A (active ingred.) | 30% | 30% | 27.3% | 30% | 30% |
| HPMC (polymeric carrier) | 40% | 40% | 36.4% | | |
| HPMC-P (HP-50) (functional carrier) | | | | 40% | 40% |
| Lactose (filler) | | 27% | | 27% | |
| Mannitol SD200 (filler) | 27% | | | | 27% |
| Celphere® SCP100 (filler) | | | 32.7 | | |
| Aerosil® 200 (filler/lubricant) | 3.0 | 3% | 3.6 | 3% | 3% |

Some of the solid dispersion granulates are further processed to tablets or capsules as final dosage form. This is achieved by blending the solid dispersion granulates with the excipients listed in Table 8 and then compressing the blended mixture into tablets (T) or filling hard gelatin capsules (H).

**TABLE 8**

| **Component** | **2F** | **2G** | **2H** | **2J** | **2K** |
|---|---|---|---|---|---|
| Solid dispersion | 83.3 mg 2A (30%) | 83.3 mg 2B (30%) | 100 mg 2C (27.3%) | 83.3 mg 2D (30%) | 333.33 mg 2B (30%) |
| Lactose (filler) | - | - | - | - | 108.167mg |
| Microcrystalline cellulose (filler) | 187.5 mg | 187.5 mg | 170.8 mg | 187.5 mg | - |
| Sodium carboxymethyl starch (disintegrant) | - | - | - | - | 35.0 mg |
| Crospovidone (disintegrant) | 10.0 mg | 10.0 mg | 10.0 mg | 10.0 mg | 15.0 mg |
| Colloidal silica (lubricant or glidant?) | - | - | - | - | 5.0 mg |
| Magnesium stearate (lubricant) | 2.2 mg | 2.2 mg | 2.2 mg | 2.2 mg | 3.5 mg |

The mass of Compound A contained in tablets/capsules 2F, 2G, 2H and 2J is 25 mg but in and 2K it is 100 mg.

The relative dissolution behaviour of some of the tablets/capsules is determined giving the results shown in TABLE 9 below. In each case the tablet/capsule is added to a solution containing 1000 mL of 0.1N HCI and 0.3% Tween® 20 , 50 rpm paddle, 37°C.

**TABLE 9**

| **Time** | **2F (%)** | **2G (%)** | **2H (%)** | **2J (%)** |
|---|---|---|---|---|
| 30 min | 71.0 ± 1.1 | 53.6 ± 1.5 | 70.0 ± 2.2 | 78.0 ± 3.6 |
| 60 min | 78.3 ± 1.1 | 55.3 ± 1.4 | 77.9 ± 1.9 | 78.7 ± 2.3 |
| 75 min | 81.4 ± 0.7 | 56.7 ± 1.1 | 83.2 ± 1.4 | 79.1 ± 2.2 |

Solid dispersions and tablets are stored at 25°C and 60% relative humidity in closed and open containers and at 40°C and 75% relative humidity in closed and open containers. However in each case no change in crystal structure is observed over 3 months as measured by differential scanning calorimetry (DSC) and X-ray powder diffraction (XRPD). This indicates that the solid dispersions remain amorphous even under accelerated conditions (i.e. 40°C/ 75% r.h. open).

### Example 3

### Melt extruded solid dispersions

Compound and A and HPMC are blended and turned into a solid dispersion by melt extrusion.

Tablets containing 25 mg amorphous Compound A as a ground solid dispersion, 187.75 mg microcrystalline cellulose, 10 mg Crospovidone® and 2.25 mg magnesium stearate are prepared in accordance with Example 1. These are melt extruded to give solid dispersions 3A and 3B. Those solid dispersions contain the components listed in Table 10 below.

**TABLE 10**

| **Component** | **3A** | **3B** |
|---|---|---|
| Compound A | 25% | 50% |
| HPMC (carrier) | 75% | 50% |

Solid dispersions and tablets are stored at 25°C and 60% relative humidity in closed and open containers and at 40°C and 75% relative humidity in closed and open containers. However in each case no change in crystal structure is observed over 3 months as measured by differential scanning calorimetry (DSC) and X-ray powder diffraction (XRPD). This indicates that the solid dispersions remain amorphous even under accelerated conditions (i.e. 40°C/ 75% r.h. open).

### Super-saturation-studies

The in-vitro dissolution of the HPMC-based system 1B and that of the enteric HPMC-P based system 2D were studied in different media and compared to the crystalline-hemihydrate or the amorphous compound.

**TABLE 11**

| | | **Compd A-amorphous** | **Component 1C** |
|---|---|---|---|
| | **Compound A (mg/ml) ~ 4mg/ml= 100% release** | | |
| **SGF** 10 min | 0.000 | 0.491 | 1.817 |
| 30 min | 0.000 | 0.000 | 1.675 |
| 60 min | 0.000 | 0.000 | 1.407 |
| 180 min | 0.000 | 0.000 | 0.000 |
| **FaSSI**F 10 min | 0.000 | 0.102 | 0.191 |
| 30 min | 0.000 | 0.063 | 0.258 |
| 60 min | 0.000 | 0.009 | 0.240 |
| 180 min | 0.000 | 0.000 | 0.048 |
| **FeSSIF** 10 min | 0.062 | 0.531 | 1.378 |
| 30 min | 0.054 | 0.575 | 1.279 |
| 60 min | 0.056 | 0.048 | 0.711 |
| 180 min | 0.044 | 0.049 | 0.086 |

| | | | |
|---|---|---|---|
| SGF:= Simulated Gastric Fluid, FaSSIF/FeSSIF:= Fasted/Fed State Simulated intestinal Fluid | | | |

In-vitro dissolution of the compound is significantly superior compared to the hemihydrate or the amorphous compound which confirms the formulation principle for this compound. In addition it is seen that the active compound has a strong tendency to precipitate rapidly upon dissolution. It is expected that this precipitated fraction has low/no bioavailability. Suprisingly, the solid dispersion system 1C clearly delays that precipitation and indicates a stabilizing effect on the dissolved compound.

**TABLE 12**

| | Component D |
|---|---|
| Compound A (mg/ml) ~ 4mg/ml= 100% release | |
| **SGF** 10 min | 0.000 |
| 30 min | 0.000 |
| 60 min | 0.000 |
| 180 min | 0.000 |
| **Buffer pH7** | |
| 10 min | 0.668 |
| 30 min | 4.173 |
| 60 min | 2.706 |
| 180 min | 1.986 |

| | |
|---|---|
| SGF:= Simulated Gastric Fluid | |

The solid dispersion system 2D made with the enteric polymer HPMC-P does not release the active compound in gastric fluid (SGF). Due to that the rapid precipitation of the compound in the gastric environment can be avoided. The system performance very well at a neutral pH at which 100% of the drug is dissolved within 30 minutes indicating enteric polymers a promising option to reduce or avoid gastric precipitation of the compound.

### Example 4

### Bioavailability studies

Material and Methods. Comparative bioavailability studies are performed according to Swiss Animal Welfare regulations, using adult male beagle dogs (stock animals weighing between 9 and 15 kg) originating either from Marshall Farms (North Rose, USA) or from Harlan (France). The dogs are fasted overnight before dosing and for an additional 5 to 6 hours after receiving their dose. The animals receive 300 to 350 g of standard pelletted dog chow every day. At least eight animals are used per dosage form, and whenever possible, dogs from the same group are re-used in subsequent experiments, using a crossover or latin square study design, in order to obtain a relative intraindividual comparison.

Dosing of capsule and tablet formulations of Compound A is performed orally by deep throat deposition, followed by a rinse with 20 mL water, to be swallowed. Blood samples are collected from a fore-leg vein (V. cephalica) into heparinized polypropylene syringes before and up to 48 hours post dose. Usually up to 12, exceptionally up to 15 blood samples are collected from each dog to obtain a precise profile of the drug levels over time. Plasma is collected after centrifugation at 2150 g, and stored frozen pending analysis. After thawing, protein precipitation and reconstitution of the samples using methanol, the samples are subjected to HPLC/tandem mass spectrometry analysis (atmospheric pressure chemical ionization interface) with a lower limit of quantification of 1.0 ng/mL.

The PK parameters after oral dosing of different tablet formulations and a control capsule give the following results (compare Table 11 below):
(a) Solid dispersion formulations reach high peak levels relatively early, with an average tmax between 1 and 1.5h, followed by a rapid decline, however, plasma levels remain close to or above the limit of quantification at 24 hours post dose, in some animals even after dosing of only 20 mg per dog.
(b) Sometimes, especially in the high dose group, a second peak was observed 24 hours after dosing of solid dispersion formulations, in most cases lower than the first peak, and in some dog individuals more pronounced than in others; the reason for this second peak is still unclear, especially since this second peak could not be reproduced in the medium dose dog group, with increased sampling points around 24 hours.
(c) Trend of non-linearity of PK was obvious, here of decreasing Cₘₐₓ/dose and AUC/dose with increasing dose: strict data comparison may only be performed within the same dose level.

Within the same dose level:
(a) Solid dispersion formulations show an about twofold higher bioavailability compared to the control capsule with micronised drug substance in a standard powder blend on one hand as well as to the melt extrusion tablet 3A with 30% drug loading on the other hand.
(b) Addition of surfactant did not show a positive effect on oral bioavailability of solid dispersion formulations in the fasted dog model.
(c) Drug loading within a range of 20-40%, the choice of the polymeric carrier (HPMC, PVP) as well as the procedure of manufacture (spray granulation, spray drying) had no relevant influence on bioavailability of the drug substance given as solid dispersion to fasted dogs.

Solid dispersion formulations are rapidly absorbed and are excellent means to increase the bioavailability of Compound A in the fasted dog model.

**TABLE 13**

| | **Nominal dose** | **Tₘₐₓ** (hr) | **Cmax** (ng/mL) | **Cmax/dose*** (ng/mL)/(mg/kg) | **AUC (0-48 hr)** (ng/mL)*h | **AUC(0-48h)/dose*** (ng/mL)*h/(mg/kg) |
|---|---|---|---|---|---|---|
| **Control** | 20 mg | 1.5 | 721 ± 546 | 385 ± 285 | 2623 ± 1584 | 1397 ± 811 |
| **1L** | 20 mg | 1.13 | 1483 ± 636 | 901 ± 321 | 5505 ± 2413 | 3361 ± 1261 |
| **1M** | 20 mg | 1.38 | 1692 ± 859 | 996 ± 438 | 6135 ± 2663 | 3627 ± 1346 |
| **1Q** | 20 mg | 1.00 | 1219 ± 779 | 723 ± 422 | 4871 ± 2394 | 2875 ± 1240 |
| **1R** | 50 mg | 1.2 | 1920 ± 995 | 384 ± 200 | 11600 ± 4180 | 2320 ± 839 |
| **IP** | 100 mg | 1.5 | 2368 ± 654 | 270 ± 81.2 | 17461 ± 10433 | 1996 ± 1170 |
| **IS** | 100 mg | 1-1.5 | 1876 ± 789 | 212 ± 85.5 | 15553 ± 10493 | 1826 ± 1375 |
| **2K** | 100 mg | 1-4 | 1981 ± 1110 | 227 ± 125 | 16327 ± 7303 | 1893 ± 876 |
| **2F** | 25 mg | 1.09 | 1440 ± 544 | 766 ± 289 | 4950 ± 1950 | 2633 ± 1037 |
| **1N** | 25 mg | 1.06 | 1620 ± 555 | 768 ± 263 | 5870 ± 2330 | 2782 ± 1104 |
| **3A** | 25 mg | 1.5 | 836 ± 516 | 398 ± 246 | 2790 ± 1540 | 1329 ± 733 |

| | | | | | | |
|---|---|---|---|---|---|---|
| *data dose-normalized to 1 mg/kg, assuming dose-linearity | | | | | | |

## Claims

1. A pharmaceutical composition in the form of a solid dispersion comprising, as active ingredient, (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a pharmaceutically acceptable salt or solvate thereof and a carrier.

2. A pharmaceutical composition according to claim 1 wherein the active ingredient is (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide semihydrate.

3. A pharmaceutical composition according to claim 1 or 2 wherein the carrier is a water soluble cellulose derivative or an enteric cellulose derivative.

4. A pharmaceutical composition according to claim 3 wherein the carrier is hydroxypropylmethylcellulose, hydroxypropylcellulose, hydroxylpropyl methylcellulose acetate succinate or hydroxypropylmethylcellulose phthalate.

5. A pharmaceutical composition according to any preceding claim further comprising one ore more surfactants, disintegrants, lubricants, glidants, antiadherents or binders.

6. A pharmaceutical composition according to claim 5 wherein the lubricant is magnesium stearate.

7. A pharmaceutical composition according to claim 5 wherein the disintegrant is sodium carboxymethyl starch or crospovidone.

8. A pharmaceutical composition according to claim 5 wherein the glidant is a colloidal silica.

9. A process for making a pharmaceutical composition according to claim 1 that comprises the steps of (a) dissolving (4R)-4-[N'-methyl-N'-(3,5-bistrifluoromethyl-benzoyl)-amino]-4-(3,4-dichlorobenzyl)-but-2-enoic acid N-[(R)-epsilon-caprolactam-3-yl]-amide or a pharmaceutically acceptable salt or solvate thereof in a solvent to form a solution; and (b) spray drying the solution to give a solid dispersion in dry powder form.

10. A blister pack comprising compartmentalised doses of a pharmaceutical composition according to claim 1.
